# EUROPEAN PATENT APPLICATION

(11) **EP 4 122 457 A1**
(43) Date of publication of application: **25.01.2023**
(21) Application number: 21382683.7
(22) Date of filing: 23.07.2021
(51) Int. Cl.: A61K 31/05, A61K 31/7048, A61K 31/718, A61P 3/06, A61K 36/185, A61K 36/63

(54) **COMPOSITION COMPRISING HYDROXYTYROSOL AND PUNICALAGIN FOR USE IN THE TREATMENT OF DISLYPIDEMIA**

(71) Applicant: Euromed, S.A., 08100 Mollet Del Vallès (Barcelona) (ES)
(72) Inventor: QUIROS FERNANDEZ, Rebeca, 18008 Granada (ES); BERMEJO LOPEZ, Laura Maria, 28981 Parla (Madrid) (ES); LOPEZ PLAZA, Bricia, 28012 Madrid (ES); ZANGARA, Andrea, 18014 Ospedaletti (IM) (IT); VILLAR GONZALEZ, Agustin, 08291 Ripollet (Barcelona) (ES)
(74) Representative: Durán-Corretjer, S.L.P.

(57) **Abstract**

The present invention refers to a composition comprising hydroxytyrosol and punicalagin for use in the treatment of dyslipidemia in a subject in need thereof.

## Description

The present invention relates to pharmaceutical compositions, and in particular it refers to a composition comprising hydroxytyrosol and punicalagin for use in the treatment of dyslipidemia in a subject in need thereof. Said composition can be in the form of a capsule administered orally.

Dyslipidemia is one of the major risk factors for the development and progression of atherosclerotic vascular disease (ASVD) and cardiovascular diseases (CVD) (Lu, H. Daugherty, A. Arter. Thromb. Vasc. Biol. 2016, 35, 485―491; Kopin, L., Lowenstein, C.J. Ann. Int. Med. 2017, 167, ITC81-ITC96).

Dyslipidemia includes a wide range of lipid abnormalities and may involve a combination of increased plasma levels of total cholesterol (TC), low-density lipoprotein cholesterol (LDL-C), and triglycerides (TG), or decreased high-density lipoprotein cholesterol (HDL-C). The prevention and sensible management of dyslipidemia can positively modify cardiovascular (CV) morbimortality (Kopin, L., Lowenstein, C.J. Ann. Int. Med. 2017, 167, ITC81-ITC96). For this reason, it is essential to find an effective solution to dyslipidemia that has few or no adverse effects and produces high adherence, in order to considerably reduce the burden of the morbimortality of ASVD and, in general, of CVD.

For the pharmaceutical industry, botanical extracts are a source of new molecules with pharmacological effects, which can be used directly and which make it possible to obtain pharmaceutical products with fewer side effects and to satisfy the growing needs for the use of natural products.

Polyphenols are becoming increasingly accepted as therapeutic substances for addressing a wide range of diseases, such as ASVD and CVD (Tejada, S.; et al., Curr. Drug Targets 2017, 18, 1477-1486; Tresserra-Rimbau, A., et al., Nutr. Metab. Cardiovasc. Dis. 2014, 24, 639-647; Santhakumar, A.B., et al., J.M. Food Chem. Toxicol. 2018, 113, 49-65; Cheng, Y.-C. et al., Oxidative Med. Cell. Longev. 2017, 2017, 1-16) and their risk factors (Tejada, S. et al., Curr. Drug Targets 2017, 18, 1477-1486; Santhakumar, A.B et al., Food Chem. Toxicol. 2018, 113, 49-65; Cheng, Y.-C. et al., Oxidative Med. Cell. Longev. 2017, 2017, 1-16; Bahramsoltani, R. et al., Crit. Rev. Food Sci. Nutr. 2017, 59, 114-132; Serino, A.; Salazar, G. Nutrients 2018, 11, 53; George, E.S. et al., Crit. Rev. Food Sci. Nutr. 2019, 59, 2772-2795). Diverse studies have reported an inverse correlation between polyphenol consumption and the risk of CV events (Tresserra-Rimbau, A., et al., Nutr. Metab. Cardiovasc. Dis. 2014, 24, 639-647; Mendonça, R.D et al., Nutr. Metab. Cardiovasc. Dis. 2019, 29, 69-78; Del Bo, C.; et al., Nutrients 2019, 11, 1355; Tangney, C.C.; Rasmussen, H.E. Rep. 2013, 15, 1-10) and overall mortality (Del Bo, C et al., Nutrients 2019, 11, 1355; Covas, M.I et al., Ann. Int. Med. 2006, 145, 333-341; Kuriyama, S et al., JAMA 2006, 296, 1255-1265; Bondonno, N.P et al., Nat. Commun. 2019, 10, 1-10).

Among these bioactive compounds, hydroxytyrosol (HT), from olives, and punicalagin (PC), from pomegranates, are noteworthy for their antioxidant, antiatherosclerotic, cardioprotective, neuroprotective, anticancer, and other effects (Yu, L.-M et al., Chem. Interact. 2019, 306, 152-162; Robles-Almazan, M. et al., Food Res. Int. 2018, 105, 654-667; Zhong, J. et al., Biochem. Biophys. Res. Commun. 2015, 467, 179-184; Chen, B. et al., Am. J. Physiol. Endocrinol. Metab. 2012, 302, E1142-E1152).

According to various *in vitro* and *in vivo* studies, the cardioprotective and antiatherosclerotic properties of HT and PC can normalize arterial prehypertension and hypertension, dyslipidemia, diabetes mellitus, oxidative and nitrative statuses, proinflammatory statuses, prothrombotic statuses, endothelial dysfunction, obesity, metabolic syndrome, and mitochondrial dysfunction, modulate the expression of cardioprotective and antiatherosclerotic genes, and reduce the adverse effects of drug treatment (Tejada, S. et al., Curr. Drug Targets 2017, 18, 1477-1486; Cheng, Y.-C. et al., Oxidative Med. Cell. Longev. 2017, 2017, 1-16; Yu, L.-M. et al., Chem. Interact. 2019, 306, 152-162; Quirós-Fernández, R. et al., Nutrients 2019, 11, 640; Robles-Imazan, M. et al., Food Res. Int. 2018, 105, 654-667; Zhong, J. et al., Biochem. Biophys. Res. Commun. 2015, 467, 179-184; Chen, B. et al., Am. J. Physiol. Endocrinol. Metab. 2012, 302, E1142-E1152; de Nigris, F. et al., Cardiovasc. Res. 2007, 73, 414-423), through multiple pathways (Tejada, S. et al., Curr. Drug Targets 2017, 18, 1477-1486; Cheng, Y.-C. et al., Oxidative Med. Cell. Longev. 2017, 2017, 1-16; Yu, L.-M. et al., Chem. Interact. 2019, 306, 152-162; Robles-Imazan, M. et al., Food Res. Int. 2018, 105, 654-667; Zhong, J. et al., Biochem. Biophys. Res. Commun. 2015, 467, 179-184; Zhang, Y. et al., J. Agric. Food Chem. 2019, 67, 13948-13959).

These natural compounds produced improvements in arterial prehypertension and hypertension, endothelial dysfunction, and high plasma circulating levels of oxidized low-density lipoprotein (oxLDL) (Quiros-Fernandez, R. et al., Nutrients 2019, 11, 640).

In this regard, the present inventors have discovered, surprisingly, that with suitable amounts of HT and PC may contribute to the reduction of the morbimortality associated with ASVD and CVD. The present inventors found the effect of an oral supplement combining these bioactive compounds on dyslipidemia in an adult population, in particular decreasing levels of LDL-C, TC and TG.

In a first aspect, the present invention discloses a composition comprising hydroxytyrosol and punicalagin for use in the treatment of dyslipidemia in a subject in need thereof.

The present inventors show the effects of the regular consumption of an oral supplement containing HT and PC on ASVD and CVD markers, such as dyslipidemia (high TC, high LDL-C, low HDL-C, and high TG), in primary prevention in an adult population.

Preferably, said hydroxytyrosol is obtained from olives.

Preferably, said punicalagin is obtained from pomegranates.

Preferably, the concentration of hydroxytyrosol in the composition is between 0.5 % and 5 % (w/w) of the total composition. More preferably, the concentration of hydroxytyrosol in the composition is between 0.5 % and 1 % (w/w) of the total composition.

Preferably, the concentration of punicalagins in the composition is between 10 % and 25 % (w/w) of the total composition. More preferably, the concentration of punicalagin in the composition is between 10 % and 20 % (w/w) of the total composition.

Preferably, the composition further comprises maltodextrin.

Preferably, the concentration of said maltodextrin is between 40 % and 85 % (w/w) of the total composition. More preferably, the concentration of said maltodextrin is between 50 % and 60 % (w/w) of the total composition.

Preferably, the composition is administered orally.

Preferably, the composition is administered in the form of a capsule.

Preferably, said composition is administered daily at least during 8 weeks.

Preferably, said dyslipidemia is an elevation of LDL-C in the subject's blood.

Preferably, said dyslipidemia is an elevation of triglycerides in the subject's blood.

The intake of three capsules daily, which contained HT (9.9 mg) and PC (195 mg), for an 8-week period significantly decreased the plasma levels of LDL-C and TG and significantly increased the HDL-C levels in an adult population with dyslipidemia without co-adjuvant treatment, and no adverse effects were observed, even though they frequently occur when using lipid-lowering drugs (e.g., myopathies, renal dysfunction, hepatic dysfunction, rhabdomyolysis, flushing, itching, gastrointestinal irritation, and stomach ulcers) (Yebyo, H.G. et al., Am. Hear. J. 2019, 210, 18-28; Sathasivam, S. Eur. J. Intern. Med. 2012, 23, 317-324; Schandelmaier, S. et al., Cochrane Database Syst. Rev. 2017, 6, CD009744; Nguyen, K.A. et al., Eur. J. Clin. Pharmacol. 2018, 74, 1099-1109; Okopień, B. et al., Expert. Rev. Clin. Pharmacol. 2018, 11, 1099-1112).

Moreover, the composition comprising HT and PC produced a significant improvement in ASVD and CVD markers, such as endothelial dysfunction, arterial prehypertension and hypertension (both systolic blood pressure (SBP) and diastolic blood pressure (DBP)) as well as circulating plasma levels of oxLDL (Quiros-Fernandez, R. et al., Nutrients 2019, 11, 640).

Furthermore, the present invention discloses a supplement capsule composed of a combination of standardized extracts (Pomalive^{®}, Euromed S.A, Mollet del Vallés, BCN, Spain) composed of 65 mg of punicalagins from a standardized pomegranate fruit extract (Pomanox^{®}, Euromed S.A., Mollet del Vallés, BCN, Spain), 3.3 mg of hydroxytyrosol from a standardized olive fruit extract (Mediteanox^{®}, Euromed S.A., Mollet del Vallés, BCN, Spain) and maltodextrin.

The present inventors found that the intake of a supplement containing HT (9.9 mg) and PC (195 mg) for 8-week improve dyslipidemia in an adult population with metabolic disorders. Therefore, the regular consumption of a supplement composed of HT and PC will reduce CV risks.

### BRIEF DESCRIPTION OF THE FIGURES

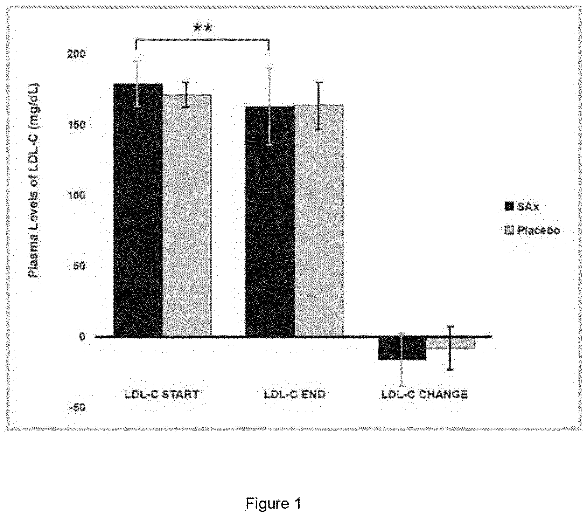

Figure 1 shows the plasma levels of low-density lipoprotein cholesterol (LDL-C) significantly decreased after SAx treatment (oral supplementation with hydroxytyrosol (HT) and punicalagin (PC)) (black colour) in subjects with high plasma levels of LDL-C, a risk factor for CVD (n = 16) (** p < 0.01). This effect was not observed after placebo treatment (grey colour). The data represent the adjusted means ± standard deviations (SDs) from multivariate models.

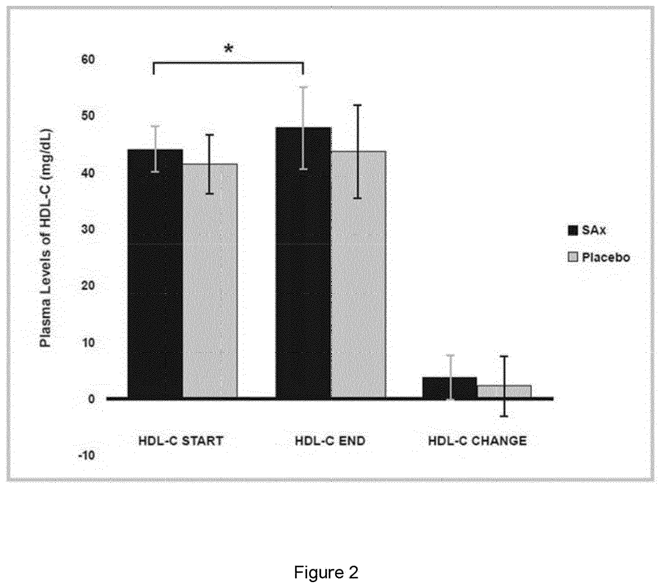

Figure 2 shows the plasma levels of high-density lipoprotein cholesterol (HDL-C) significantly increased after SAx treatment (oral supplementation with hydroxytyrosol (HT) and punicalagin (PC)) (black colour) in subjects with low plasma levels of HDL-C, a risk factor for CVD (n = 8) (* p < 0.05). This effect was not observed after placebo treatment (grey colour). The data represent the adjusted means ± standard deviations (SDs) from multivariate models.

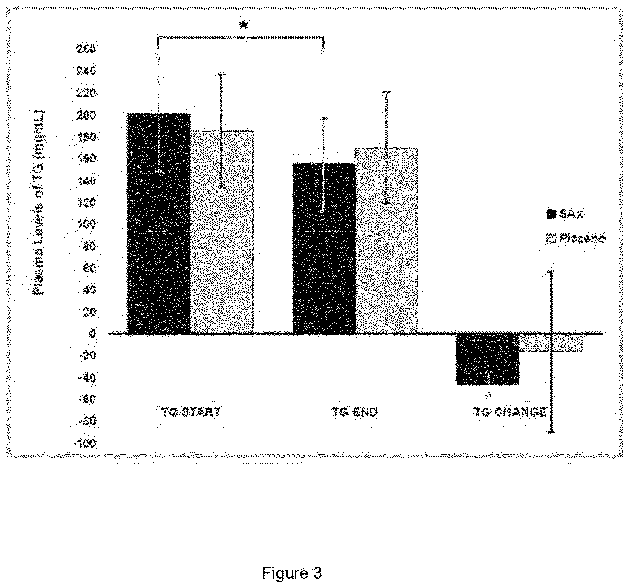

Figure 3 shows the plasma levels of triglycerides (TG) significantly decreased following SAx treatment (oral supplementation with hydroxytyrosol (HT) and punicalagin (PC))) (black colour) in subjects with hypertriglyceridemia, a risk factor for CVD (n = 4) (* p < 0.05). This effect was not observed after placebo treatment (grey colour). The data represent the adjusted means ± standard deviations (SDs) from multivariate models.

Unless defined otherwise, all technical and scientific terms used herein have the same meaning as commonly understood by one of ordinary skill in the art pertinent to the compositions described. As used herein, the following terms and phrases have the meanings ascribed to them unless specified otherwise.

The terms "a," "an," and "the" include plural referents, unless the context clearly indicates otherwise.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Unless otherwise defined, all technical and scientific terms used herein have the same meaning as commonly understood by one of ordinary skill in the art to which this invention pertains. Exemplary compositions are described below, although compositions similar or equivalent to those described herein can also be used and will be apparent to those of skill in the art. All publications and other references mentioned herein are incorporated by reference in their entirety. In case of conflict, the present specification, including definitions, will control. The compositions and examples are illustrative only and not intended to be limiting.

Each embodiment in this specification is to be applied mutatis mutandis to every other embodiment unless expressly stated otherwise.

The following terms, unless otherwise indicated, shall be understood to have the following meanings:

The term "treatment" or "treating" means any treatment of a disease or disorder in a subject, such as a mammal, including: preventing or protecting against the disease or disorder, that is, causing the clinical symptoms not to develop; inhibiting the disease or disorder, that is, arresting or suppressing the development of clinical symptoms; and/or relieving the disease or disorder that is, causing the regression of clinical symptoms. In some embodiments, the term treatment is used for the treatment of dyslipidemia.

In the present invention the term "dyslipidemia" refers to an abnormal amount of lipids (e.g. triglycerides, cholesterol and/or fat phospholipids) in the blood. In developed countries, most dyslipidemias are hyperlipidemias; that is, an elevation of lipids in the blood. This is often due to diet and lifestyle.

In the present invention the term "hydroxytyrosol" or "HT" refers to a phenylethanoid, a type of phenolic phytochemical with antioxidant properties *in vitro.* In nature, hydroxytyrosol is found in olive leaf and olive oil, in the form of its elenolic acid ester oleuropein and, especially after degradation, in its plain form.

In the present invention the term "punicalagin" or "PC" refers to an ellagitannin, a type of phenolic compound. It is found in forms alpha and beta in pomegranates *(Punica granatum),* in *Terminalia catappa* and *Terminalia myriocarpa,* and in *Combretum molle,* the velvet bushwillow, a plant species found in South Africa.

The example is provided below to illustrate, but not limit, the invention.

### Examples

### EXAMPLE 1: Clinical trial of the present invention

The present clinical trial was performed between February and June 2013. This study involved 84 apparently healthy subjects (17 males (20.2%) and 67 females (79.8%)) who were found to be suitable for inclusion. There were 17 participants subsequently lost to follow-up (nine in the SAx/Placebo sequence and eight in the Placebo/SAx sequence) for personal reasons (n = 15) and noncompliance with the treatment instructions (n = 2). As such, 67 participants (14 males (20.9%) and 53 females (79.1%)) completed the present 20-week clinical trial; only their results were included in the statistical analyses.

The treatment sequences consisted of an oral supplementation containing HT and PC (SAx: 9.9 mg of HT, 195 mg of PC, and 995.1 mg of maltodextrin per day) or a placebo (1200 mg of maltodextrin per day) during an 8-week period, followed by a 4-week washout interval and finally followed by a crossover.

The participants ingested three capsules per day (SAx or placebo) with meals during the 8-week intervention period. Throughout the clinical trial, the researchers and participants did not know the treatment sequence to which the participants had been assigned, and they remained blinded until the end of the study.

Each supplement capsule (SAx) was composed of 65 mg of PC from a standardized pomegranate fruit extract (Pomanox^{®}, Euromed S.A., Mollet del Vallés, BCN, Spain), 3.3 mg of HT from a standardized olive fruit extract (Mediteanox^{®}, Euromed S.A., Mollet del Vallés, BCN, Spain), and 331.7 mg of maltodextrin.

Each placebo capsule was composed of 400 mg of maltodextrin (Euromed S.A., Mollet del Vallés, BCN, Spain). Both the supplement capsules (with specific concentrations of HT and PC) and placebo capsules were specially prepared for this clinical trial. They were packaged in 15-unit blister packs, which were labeled as L1 or L2 to preserve the blinded conditions. The participants were provided with as many capsules as necessary to complete the 8-week intervention period (SAx or placebo) during recurring scheduled visits.

With regard to the baseline state in the present clinical trial, there were no significant differences between the participants assigned to the different intervention sequences (Placebo/SAx and SAx/Placebo) in gender, age, smoking habits, anthropometry, lipid profiles, or other variables (Table 1). The average age of the population was 53.0 ± 4.5 years. The average BMI was 24.6 ± 3.1 kg/m2.

**Table 1. Baseline characteristics of the participants.**

| | | **Placebo/SAx (*n*** = **33)** | **SAx/Placebo (*n*** = **34)** |
|---|---|---|---|
| Gender | (Female %, *n*) | 78.79 (26) | 79.41 (27) |
| Age | (years) | 53.21 ± 4.2 | 52.79 ± 4.8 |
| Smoking | (Smokers %, *n*) | 18.18 (6) | 26.47 (9) |
| Weight | (kg) | 66.26 ± 11.8 | 64.08 ± 10.9 |
| BMI | (kg/m²) | 24.64 ± 2.9 | 24.56 ± 3.2 |
| Waist circumference | (cm) | 80.51 ± 9.2 | 82.58 ± 9.8 |
| FM | (%) | 29.18 ± 6.7 | 28.76 ± 6.4 |
| FFM | (%) | 70.82 ± 6.7 | 71.24 ± 6.4 |
| MM | (%) | 48.03 ± 7.7 | 47.87 ± 5.5 |
| SBP | (mmHg) | 110.3 ± 13.1 | 110.9 ± 12.9 |
| DBP | (mmHg) | 74.06 ± 10.8 | 73.75 ± 9.5 |
| HR | (bpm) | 67.36 ± 8.9 | 70.41 ± 7.5 |
| TC | (mg/dL) | 226.7 ± 29.6 | 224.6 ± 35.4 |
| LDL-C | (mg/dL) | 144.3 ± 23.9 | 145.3 ± 28.6 |
| HDL-C | (mg/dL) | 66.25 ± 12.9 | 62.00 ± 12.6 |
| TG | (mg/dL) | 80.56 ± 24.6 | 86.52 ± 44.0 |

Data presented as means ± standard deviations (SDs). SAx: oral supplementation with hydroxytyrosol (HT) and punicalagin (PC); BMI: body mass index; FM: fat mass; FFM: fat-free mass; MM: muscle mass; SBP: systolic blood pressure; DBP: diastolic blood pressure; HR: heart rate; TC: total cholesterol; LDL-C: low-density lipoprotein cholesterol; HDL-C: high-density lipoprotein cholesterol; and TG: triglycerides. There were no significant differences in the baseline state between the two intervention sequences.

Regarding the results for the dietary and anthropometric variables compared between the beginning and end of the different intervention periods, no significant differences were observed, nor were significant differences found between the different periods in terms of changes in these variables (Table 2).

**Table 2. The dietary and anthropometric variables at the beginning and end of the supplementation and placebo periods.**

| | | | **SAx (*n* = 67)** | **Placebo (*n* = 67)** |
|---|---|---|---|---|
| Energy | (kcal/day) | Start | 1923 ± 513.6 | 1864 ± 471.9 |
| | | End | 1891 ± 549.4 | 1881 ± 569.5 |
| | | Change | -31.88 ± 463.2 | 17.07 ± 355.5 |
| Carbohydrates | (%) | Start | 38.06 ± 6.5 | 38.46 ± 8.9 |
| | | End | 37.62 ± 6.3 | 39.06 ± 6.5 |
| | | Change | -0.439 ± 5.7 | 0.598 ± 8.1 |
| Proteins | (%) | Start | 17.24 ±3.7 | 17.44 ± 2.9 |
| | | End | 17.43 ± 3.6 | 17.60 ± 3.1 |
| | | Change | 0.193 ± 4.4 | 0.164 ± 3.5 |
| Lipids | (%) | Start | 41.42 ± 6.3 | 40.48 ± 8.8 |
| | | End | 41.36 ± 5.7 | 40.19 ± 5.9 |
| | | Change | -0.067 ± 5.3 | -0.287 ± 8.0 |
| SFA | (%) | Start | 12.33 ± 2.9 | 12.46 ± 4.0 |
| | | End | 12.38 ± 2.8 | 12.03 ± 2.9 |
| | | Change | 0.049 ± 2.7 | -0.433 ± 4.2 |
| MUFA | (%) | Start | 19.10 ± 3.6 | 18.82 ± 4.9 |
| | | End | 19.56 ± 4.0 | 19.29 ± 3.5 |
| | | Change | 0.453 ± 3.8 | 0.470 ± 4.2 |
| PUFA | (%) | Start | 6.45 ± 2.3 | 5.60 ± 1.6 |
| | | End | 5.98 ± 1.7 | 5.46 ± 1.8 |
| | | Change | -0.470 ± 2.4 | -0.134 ± 1.4 |
| Total Cholesterol | (mg/dL) | Start | 350.5 ± 172.8 | 303.9 ± 150.4 |
| | | End | 328.1 ± 133.8 | 323.0 ± 115.9 |
| | | Change | -22.35 ± 210.8 | 19.04 ± 130.1 |
| Fiber | (g/d) | Start | 21.95 ± 7.8 | 21.71 ± 8.0 |
| | | End | 21.68 ± 8.9 | 20.47 ± 7.3 |
| | | Change | -0.275 ± 8.2 | -1.234 ± 6.5 |
| Weight | (kg) | Start | 65.10 ± 11.3 | 65.10 ± 11.2 |
| | | End | 64.93 ± 11.2 | 64.85 ± 11.3 |
| | | Change | -0.173 ± 1.3 | -0.249 ± 1.0 |
| BMI | (kg/m²) | Start | 24.58 ± 3.0 | 24.63 ± 3.0 |
| | | End | 24.51 ± 3.0 | 24.48 ± 3.0 |
| | | Change | -0.068 ± 0.5 | -0.151 ± 0.6 |
| Waist Circumference | (cm) | Start | 81.85 ± 9.0 | 81.24 ± 9.8 |
| | | End | 81.82 ± 9.6 | 81.25 ± 9.6 |
| | | Change | -0.034 ± 2.9 | 0.008 ±3.7 |
| FM | (%) | Start | 29.16 ± 6.6 | 28.90 ± 6.5 |
| | | End | 29.56 ± 6.8 | 29.79 ± 7.2 |
| | | Change | 0.400 ± 2.8 | 0.891 ± 3.7 |
| FFM | (%) | Start | 70.84 ± 6.6 | 71.10 ± 6.5 |
| | | End | 70.44 ± 6.8 | 70.21 ± 7.2 |
| | | Change | -0.400 ± 2.8 | -0.891 ± 3.7 |
| MM | (%) | Start | 47.63 ± 6.2 | 47.52 ± 6.5 |
| | | End | 46.67 ± 5.7 | 46.44 ± 5.8 |
| | | Change | -0.970 ± 5.1 | -1.082 ± 5.8 |

Data expressed as means ± standard deviations (SDs). SAx: oral supplementation with hydroxytyrosol (HT) and punicalagin (PC); SFA: saturated fatty acids; MUFA: monounsaturated fatty acids; PUFA: polyunsaturated fatty acids; BMI: body mass index; FM: fat mass; FFM: fat-free mass; and MM: muscle mass. In the present clinical trial, no significant differences were observed between the beginning and end of the different intervention periods or in the changes.

Table 3 shows the values obtained for the lipid-profile variables examined. A significant reduction after SAx treatment was observed in LDL-C plasma levels in subjects with initially high LDL-C plasma levels (≥160 mg/dL) (SAx period start: 179.13 ± 16.18; SAx period end: 162.93 ± 27.05 mg/dL; p < 0.004). This significant effect did not occur in the subjects following placebo treatment (Placebo period start: 171.63 ± 9.08; Placebo period end: 163.56 ± 16.87 mg/dL; p < 0.079). The results are also shown in Figure 1.

**Table 3. Lipid-profile variables at the beginning and end of the supplementation and placebo periods in population with dyslipidemia.**

| | | **SAx (*n* = 67)** | **Placebo (*n* = 67)** |
|---|---|---|---|
| TC | (mg/dL) | Start 237.6 ± 26.0 | 238.4 ± 20.0 |
| | (*n* = 49) | End 234.9 ± 25.1 | 233.0 ± 22.8 |
| | | Change -2.776 ± 18.8 | -5.388 ± 18.8 |
| LDL-C | | Start 179.1 ± 16.2 | 171.6 ± 9.1 |
| | (mg/dL) (*n* = 16) | End 162.9 ± 27.1 ** | 163.6 ± 16.9 |
| | | Change -16.20 ± 18.5 | -8.063 ± 15.1 |
| HDL-C | (mg/dL) | Start 44.25 ± 4.0 | 41.50 ± 5.2 |
| | (*n* = 8) | End 48.00 ± 7.3 * | 43.75 ± 8.3 |
| | | Change 3.750 ± 4.0 | 2.250 ± 5.4 |
| TG | (mg/dL) | Start 200.7 ± 51.4 | 186.0 ± 51.5 |
| | (*n* = 4) | End 155.3 ± 42.4 * | 170.5 ± 50.3 |
| | | Change -45.33 ± 10.5 | -15.50 ± 73.1 |

Data expressed as means ± standard deviations (SDs). SAx: oral supplementation with hydroxytyrosol (HT) and punicalagin (PC); TC: total cholesterol; LDL-C: low-density lipoprotein cholesterol; HDL-C: high-density lipoprotein cholesterol; and TG: triglycerides. In the present clinical trial, significant differences were observed between the beginning and end of the SAx period (* *p* < 0.05, ** *p<* 0.01). There were no significant differences in the placebo period or in the changes in the different intervention periods.

In addition, at the end of the placebo period, a significant decrease of HDL-C in the plasma levels was observed in the total population (Placebo period start: 64.49 ± 12.65; Placebo period end: 62.55 ± 11.57 mg/dL; p < 0.016). After the SAx period, a significant increase of HDL-C in the plasma levels was observed in subjects with initially low HDL-C plasma levels (<50 mg/dL) (SAx period start: 44.25 ± 3.99; SAx period end: 48.00 ± 7.27 mg/dL; p < 0.033). After the placebo period, this significant effect on HDL-C plasma levels was not observed in the subjects (Placebo period start: 41.50 ± 5.19; Placebo period end: 43.75 ± 8.26 mg/dL; p < 0.464). The results are also shown in Figure 2. In the present clinical trial, only women showed low HDL-C plasma levels. There were no men who presented low HDL-C plasma levels.

At the end of the SAx period, a significant decrease of TG in the plasma levels was observed in subjects with hypertriglyceridemia (≥150 mg/dL) (SAx period start: 200.67 ± 51.38; SAx period end: 155.33 ± 42.44 mg/dL; p < 0.017). This significant effect of TG on the plasma levels was not present after the placebo period in the subjects (Placebo period start: 186.00 ± 51.54; Placebo period end: 170.50 ± 50.32 mg/dL; p < 0.700). The results are also shown in Figure 3.

## Claims

1. Composition comprising hydroxytyrosol and punicalagin for use in the treatment of dyslipidemia in a subject in need thereof.

2. Composition for use, according to claim 1, wherein said hydroxytyrosol is obtained from olives.

3. Composition for use, according to claim 1, wherein said punicalagin is obtained from pomegranates.

4. Composition for use, according to any of the preceding claims, wherein the concentration of hydroxytyrosol in the composition is between 0.5 % and 5 % (w/w) of the total composition.

5. Composition for use, according to claim 4, wherein the concentration of hydroxytyrosol in the composition is between 0.5 % and 1 % (w/w) of the total composition.

6. Composition for use, according to any of the preceding claims, wherein the concentration of punicalagins in the composition is between 10 % and 25 % (w/w) of the total composition.

7. Composition for use, according to claim 6, wherein the concentration of punicalagins in the composition is between 10 % and 20 % (w/w) of the total composition.

8. Composition for use, according to any of the preceding claims, further comprises maltodextrin.

9. Composition for use, according to claim 8, wherein the concentration of said maltodextrin is between 40 % and 85 % (w/w) of the total composition.

10. Composition for use, according to claim 9, wherein the concentration of said maltodextrin is between 50 % and 60 % (w/w) of the total composition.

11. Composition for use, according to any of the preceding claims, wherein said composition is administered orally.

12. Composition for use, according to any of the preceding claims, wherein said composition is administered in the form of a capsule.

13. Composition for use, according to any of the preceding claims, wherein said composition is administered daily at least during 8 weeks.

14. Composition for use, according to any of the preceding claims, wherein said dyslipidemia is an elevation of LDL-C in the subject's blood.

15. Composition for use, according to any of the preceding claims, wherein said dyslipidemia is an elevation of triglycerides in the subject's blood.
